# EUROPÄISCHE PATENTANMELDUNG

(11) **EP 1 923 967 A2**
(43) Veröffentlichungstag der Anmeldung: **21.05.2008**
(21) Anmeldenummer: 07020387.2
(22) Anmeldetag: 18.10.2007
(51) Int. Cl.: H01R 24/04, H01R 13/33, A61N 1/375

(54) **Kontaktanordnung, Kontaktbaugruppe, implantierbare Vorrichtung und Elektrodenleitung**

(30) Priorität: 15.11.2006 DE 102006053729
(71) Anmelder: BIOTRONIK CRM Patent AG, 6341 Baar (CH)
(72) Erfinder: Rebentisch, Ronald, 10967 Berlin (DE)
(74) Vertreter: Lindner-Vogt, Karin L.

(57) **Zusammenfassung**

Die vorliegende Erfindung betrifft eine Kontaktanordnung zur Herstellung einer elektrisch leitenden Verbindung zwischen einem im Wesentlichen zylindrischen Stecker und einer im Wesentlichen hohlzylindrischen Buchse, vorzugsweise für die Verbindung einer Elektrode mit einer elektronischen Vorrichtung eines implantierbaren Geräts, bzw. eine Kontaktbaugruppe für einen Stecker oder eine Buchse mit einer oder mehreren solcher Kontaktanordnungen. Die Kontaktanordnung bildet entweder einen Kontaktabschnitt des Steckers oder einen Kontaktabschnitt der Buchse. An dem Buchsenkontaktabschnitt ist eine Öffnung vorgesehen, in die der Stecker einführbar ist. Die Kontaktanordnung weist mindestens ein Kontaktelement auf, wobei das Kontaktelement aus einem Geflecht mit Fasern besteht, die metallische Leitfähigkeit besitzen. Mindestens ein Ende einer Vielzahl von Fasern des Fasergeflechts des Kontaktelements ragt in die Öffnung bzw. ragt von der Mantelfläche nach außen weg. Die Erfindung betrifft außerdem eine implantierbare Vorrichtung mit einer Buchsen-Kontaktbaugruppe und einer elektronischen Vorrichtung mit mindestens einem Anschluss, wobei der mindestens eine Anschluss der elektronischen Vorrichtung mit einer Kontaktanordnung elektrisch leitend verbunden ist, bzw. eine Elektrodenleitung mit einem an einem Ende angeordneten Stecker mit einer Stecker-Kontaktbaugruppe, wobei die Elektrode elektrisch leitend mit einer Kontaktanordnung verbunden ist. Der Vorteil der Erfindung besteht darin, dass die Kontaktanordnung einen hohen Grad an Redundanz bei der Kontaktierung und eine hohe Kontaktstabilität unter mechanischer Last aufweist. Außerdem wird Reibkorrosion vermieden.

## Beschreibung

Die vorliegende Erfindung betrifft eine Kontaktanordnung zur Herstellung einer elektrisch leitenden Verbindung zwischen einem im Wesentlichen zylindrischen Stecker und einer im Wesentlichen hohlzylindrischen Buchse, vorzugsweise für die Verbindung einer Elektrode mit einer elektronischen implantierbaren Vorrichtung, wobei die Kontaktanordnung entweder den Kontaktabschnitt des Steckers oder den Kontaktabschnitt der Buchse bildet, wobei der Buchsenkontaktabschnitt eine Öffnung aufweist, in die der Stecker einführbar ist, wobei die Kontaktanordnung mindestens ein Kontaktelement aufweist, wobei das Kontaktelement aus einem Geflecht mit Fasern besteht, die metallische Leitfähigkeit besitzen. Die Erfindung betrifft ferner eine Kontaktbaugruppe, eine implantierbare Vorrichtung sowie eine Elektrodenleitung.

Im medizinischen Bereich werden häufig implantierbare Vorrichtungen eingesetzt, welche mit elektronischen Einrichtungen, bspw. Leiterplatten oder dergleichen versehen sind. Elektronische Einrichtungen wie Herzschrittmacher (pacemaker), Defibrillatoren oder neurologische Geräte wie Hirnschrittmacher für tiefe Hirnstimulation (deep brain stimulation), Rückenmarkstimulationsgeräte, TENS (transcutaneous electrical nerve stimulator) oder Geräte für muskuläre Stimulationstherapie sowie Diagnosegeräte, welche die chemischen Eigenschaften des Blutes des Patienten oder anderer Körperteile oder andere Körpereigenschaften untersuchen, verwenden Elektroden, die durch den Körper des Patienten geführt werden und mit der implantierbaren Vorrichtung elektrisch leitend verbunden werden. Die implantierbare elektronische Vorrichtung weist hierfür eine im Wesentlichen buchsenförmige oder hohlzylindrische Anschlussvorrichtung (Buchse, Kontaktstift) in Form einer Kontaktbaugruppe auf, in welche der Stecker der Elektrodenleitung eingeführt wird, um eine elektrisch leitende Verbindung zwischen der Elektrode und der in der implantierbaren Vorrichtung angeordneten elektronischen Vorrichtung herzustellen. Die Anschlussvorrichtung wird auch als Header bezeichnet. Ebenso ist an der Elektrodenleitung, welche die Elektrode aufweist, ein im Wesentlichen zylindrischer Stecker mit einer Kontaktbaugruppe vorgesehen, die in die Buchse eingeführt wird, um die elektrisch leitende Verbindung herzustellen.

Zukünftig werden verstärkt mehrpolige Stecker mit mehr als zwei Polen eingesetzt werden. Beispielsweise ist der Stecker nach der Norm IS-4 ein 4-poliger Stecker, der eine im Wesentlichen zylindrische Form aufweist und vier elektrisch leitende Kontaktabschnitte, die in axialer Richtung nacheinander angeordnet sind, aufweist.

In der US 4,469,104 ist ein im Wesentlichen zylindrischer Stecker angegeben, welcher in eine Buchse eingeführt werden kann. Hierfür weist die Buchse eine im Wesentlichen zylindrische Öffnung auf. An dem multipolaren Stecker sind drei in Richtung der Längsachse hintereinander angeordnete Kontaktanordnungen vorgesehen, welche jeweils durch isolierende Bereiche von einander getrennt sind. In einem mit einer Nut versehenen Halteelement ist ein leitfähiger Ring in Form eines gewebten Metall-Toroids angeordnet. Der Toroid ist aus einem blattförmigen gewebten Metall hergestellt. Nachdem der Toroid durch Einrollen des blattförmigen Materials geformt ist, wird das Material an seinen Enden zusammen geschweißt, um einen geschlossenen elastischen Ring zu bilden. Der leitfähige Ring ist derart in dem Halteelement angeordnet, dass er ein wenig über die Mantelfläche der angrenzenden Bereiche des Steckers hinaus ragt. Nach dem Einstecken des Steckers in die Öffnung der Kontaktbaugruppe berührt der leitfähige Ring die an der Buchse angeordneten Kontaktabschnitte, die jeweils die Form eines Metallrings haben. Hierdurch wird eine elektrisch leitende Verbindung zwischen der jeweiligen Kontaktanordnung an dem Stecker und dem Kontaktabschnitt der Buchse hergestellt, um die Elektrode mit der elektronischen Vorrichtung in dem implantierbaren Gerät elektrisch leitend zu verbinden.

In Druckschrift WO 2005/014108 wird eine Kontaktanordnung für die Verbindung einer Elektrode und eines implantierbaren medizinischen Gerätes beschrieben. Die Kontaktanordnung weist einen Verbindungsclip auf, der aus einem einzigen Draht besteht und derart geformt ist, dass er einen ersten Federarm und einen zweiten Federarm aufweist. Beide Federarme grenzen an einen oberen gekrümmten Abschnitt, der den oberen Abschnitt des Verbindungsclip bildet. Die Federarme weisen im Wesentlichen gerade Seitenabschnitte auf, welche von dem oberen gekrümmten Abschnitt zu einem unteren gekrümmten Abschnitt reichen. In dem Gehäuse der Kontaktanordnung mit durchgehender Öffnung ist der Verbindungsclip in einer teilweise aufgebogenen Position angeordnet. Der Verbindungsclip ist derart in dem Gehäuse der Kontaktanordnung angeordnet, dass er in radialer Richtung nach innen ein wenig aus dem Gehäuse vorsteht. Das Gehäuse ist hierbei so geformt, dass nach dem Einführen des Steckers in die Öffnung der Kontaktanordnung der Verbindungsclip weiter in radialer Richtung nach außen gedrückt wird, so dass eine Verbindung zwischen Stecker und elektronischer Einrichtung hergestellt wird.

Aus Dokument EP 1 062 986 B1 ist ein implantierbares elektronisches Gerät mit einer Verriegelungseinrichtung für Stecker und eine mit dem Gerät verbindbaren Elektrodenleitung bekannt. Das elektronische Gerät weist eine im Wesentlichen buchsenförmige Anschlussvorrichtung für den Stecker der Elektrodenleitung mit der Verriegelungseinrichtung auf, welche aus einer manuell betätigbare, exzentrisch gelagerten Spannnocke und einem in der Anschlussvorrichtung vorgesehenen Kontaktelement für den differenten Pol des Steckers einer mit dem Herzschrittmacher zu verbindenden koaxialen Elektrodenleitung gebildet ist. Die exzentrisch gelagerte Spannnocke steht an ihrem Rand mit dem Kontaktelement in Wirkungseingriff, so dass eine Drehbewegung der Spannnocke auf das Kontaktelement übertragen wird. Dadurch wird das den differenten Pol des Steckers formschlüssig umgreifende Kontaktelement derart reversibel verformt, dass zwischen dem differenten Pol und dem Kontaktelement eine kraftschlüssige Verbindung entsteht, welche den Stecker innerhalb der Anschlussvorrichtung fixiert und gleichzeitig einen guten galvanischen Kontakt zwischen dem Kontaktelement und dem differenten Pol des Steckers gewährleistet.

In Druckschrift WO 2005/105207 A2 ist ein elektrischer Verbinder, insbesondere ein medizinisch implantierbarer elektrischer Verbinder mit einem Gehäuse und einer durchgehenden Bohrung bekannt. In einer Nut des Gehäuses ist eine Feder angeordnet, welche dazu dient, eine Beschichtung (typischerweise eine Oxidschicht) von der Oberfläche der Nut und der Steckeroberfläche abzuschaben, so dass ein reduzierter Widerstand und eine reduzierte Widerstandsvariabilität erreicht wird. Insbesondere ist die Feder als schräg liegende spulenartige Feder ausgebildet.

Ebenfalls ein elektrischer Kontakt mit einem Federring ist aus der Druckschrift US 2006/0047322 A1 bekannt. Diese Druckschrift zeigt eine Kontaktbaugruppe mit einer Vielzahl von Kontaktanordnungen mit elektrisch leitenden Federringen, welche in entsprechenden Vertiefungen der Kontaktanordnungen vorgesehen sind.

In Druckschrift US 4,995,389 ist eine Kontaktbaugruppe beschrieben, die eine Vielzahl von Kontaktanordnungen mit ringförmigen Federkontakten aufweist.

Eine Kontaktanordnung zum Anschluss einer Elektrodenleitung an ein implantierbares Gerät ist aus Druckschrift DE 10 2004 017 659 A1 bekannt. Die Kontaktanordnung weist eine elektrische Anschlussbuchse mit einer Buchsen-Längsachse und wenigstens einer Öffnung zum Einführen eines Steckers entlang der Buchsen-Längsachse auf. Als Kontaktelement ist eine einstückige, metallische Federkontaktzunge mit quer zur Federauslenkungsrichtung verlaufenden Federkontaktzungen-Abschnitten vorgesehen, welche durch Wendeabschnitte miteinander verbunden sind. Die Federkontaktzunge ist insbesondere derart gewendet, dass sie eine Wellenform, beispielsweise eine S-förmige Wellenform aufweist.

Ebenfalls ein Kontaktelement aus einer einstückigen, metallischen Federkontaktzunge ist in der Druckschrift US 2005/0261745 A1 angegeben. Mehrere Kontaktelemente sind hierbei derart gebogen, dass ihr Endabschnitt in die Öffnung hineinreicht und eine Tangente an einen imaginären Kreis bildet, der einen geringeren Durchmesser als die Öffnung der Elektrodenanordnung für den Stecker aufweist. In dieser Druckschrift wird auch noch eine zweite Variante einer Kontaktanordnung beschrieben, bei welcher die Kontaktelemente aus stangenförmigen Elementen bestehen, deren Endabschnitte von der Öffnung für den Stecker weg gerichtet sind. Analog zu den blattförmigen Elementen sind die stangenförmigen Elemente derart gebogen, dass sie eine Tangente an einen imaginären Kreis bilden, der einen kleineren Durchmesser aufweist als der Querschnitt der Steckeröffnung.

Eine weitere Möglichkeit für eine Kontaktanordnung wird in der Druckschrift US 2002/0128692 A1 offenbart. Die Kontaktanordnung enthält ringförmige Federelemente, in denen kreisförmige Öffnungen vorgesehen sind. In diesen kreisförmigen Öffnungen sind kugelförmige Kontaktelemente angeordnet, die durch die Federwirkung der Feder in Richtung weg von der Öffnung gedrückt werden, jedoch in diese etwas nach innen vorstehen, so dass zwischen dem eingeführten Stecker und den Metallkugeln ein Kontakt hergestellt wird.

Für die Herstellung obiger bekannter Kontaktanordnungen mit metallischen Federelementen oder -zungen sind hoch präzise und damit kostenintensive Fertigungseinrichtungen erforderlich. Üblicherweise werden diese Kontaktelemente über einen Stanz-Biege-Prozess in einem Folgeverbundwerkzeug aus einem Bandmaterial hergestellt. Beispielsweise wird ein rechteckiger Blechstreifen erzeugt, der über die längere Seite so umgeformt wird, dass er zur Mantelfläche eines nicht ganz geschlossenen Rohres wird. Indem aus dieser Mantelfläche Segmente in Richtung der kürzeren Seite herausgestanzt werden, entsteht eine bestimmte Anzahl von Stegen. Diese Stege werden auf halber Höhe der Mantelfläche so umgeformt, dass sie gleichmäßig radial nach innen ragen und so eine Einschnürung in der Mantelfläche bilden, über die dann später die elektrische Kontaktierung erfolgen kann. Ein derartiges Kontaktelement hat jedoch keinen definierten Radius, über den sich eine Kontaktkraft auf einen zylindrischen Stecker einstellen ließe. Erst wenn ein Kontaktelement unter radialer Vorspannung in die Bohrung einer formstabilen Hülse eingeführt wird, entsteht daraus eine funktionstüchtige Kontaktbaugruppe.

Die in Alltagselektrogeräten gängigen und damit auch preiswerten elektrischen Kontaktwerkstoffe können bei Langzeitimplantaten nicht zum Einsatz kommen, da neben dem eher allgemeinen Kriterium der Korrosionsstabilität auch das Kriterium der Biokompatibilität zu erfüllen ist. Für nicht häufig verwendete Werkstoffe kann aber die für eine laufende Fertigung erforderliche ständige Verfügbarkeit der Materialien problematisch werden.

Für einen stabilen elektrischen Kontakt einer steckbaren Verbindung ist zunächst eine makroskopische Mindestandruckkraft zwischen den Kontaktpartnern Stecker und Buchse erforderlich. Diese Mindestandruckkraft für einen einzelnen Kontakt hängt von den verwendeten Werkstoffen ab, in erster Näherung kann eine Normalkraft von größer als 1 N angesetzt werden. Wird eine derartige Mindestandruckkraft nicht erreicht, so können zum einen elektrisch isolierende Deckschichten der Werkstoffoberflächen im Kontaktbereich nicht entfernt werden und es ist zum anderen die Langzeitstabilität der elektrischen Kontaktierung nicht gewährleistet.

Bei der konstruktiv mechanischen Auslegung eines Steckkontaktsystems muss aber auch die Steckbarkeit gewährleistet sein. Ein Parameter hierfür ist die maximal zulässige Einschubkraft. Die Federsteifigkeit der Kontaktelemente kann nicht beliebig erhöht werden, um so zu einer besseren elektrischen Kontaktsicherheit zu gelangen, da sonst Probleme bei dem Steckvorgang auftreten können.

Für einen stabilen elektrischen Kontakt ist es demnach notwendig, dass die beim Einführen des zylindrischen Steckers (Kontaktstift) aufgebaute mechanische Spannung so groß ist, dass im Kontaktbereich störende, elektrisch isolierende Deckschichten entfernt werden, so dass die Kontaktpartner in direkten metallischen Kontakt gelangen können. Für eine zeitliche Konstanz des elektrischen Kontaktwiderstands muss der so hergestellte Kontaktbereich über die gesamte Dauer der Kontaktierung metallisch leitend bleiben. Es ist demnach konstruktiv sicherzustellen, dass ein solcher Kontakt bei schwacher mechanischer Vorspannung und unter radialer mechanischer Last nicht abheben (atmen) kann. Es ist aber auch zu gewährleisten, dass ein derartiger Kontakt bei starker mechanischer Vorspannung und axialer mechanischer Last nicht zu reiben beginnt und es damit zur Reibkorrosion (Fretting) kommen kann.

Aufgabe der vorliegenden Erfindung ist es daher, eine Kontaktanordnung zu schaffen, welche einen hohen Grad an Redundanz bei der Kontaktierung und eine hohe Kontaktstabilität unter mechanischer Last aufweist. Außerdem soll Reibkorrosion vermieden werden. Eine analoge Aufgabenstellung besteht auch im Hinblick auf die Kontaktbaugruppe, die implantierbare Vorrichtung und die Elektrodenleitung.

Erfindungsgemäß wird diese Aufgabe durch eine Kontaktanordnung gelöst, bei welcher mindestens ein Ende einer Vielzahl von Fasern des Fasergeflechts des Kontaktelements in die Öffnung ragt bzw. von der Mantelfläche nach außen wegragt.

Die Erfindung beruht auf der Erkenntnis, dass nur ein Bruchteil der mechanisch durch Andruck hergestellten Kontaktfläche überhaupt für den elektrischen Kontakt wirksam wird. Die elektrisch wirksamen, bei indirektem metallischen Kontakt stehenden Flächen werden als "a-spots" oder "contact-spots" bezeichneet (P. van Dijk, 'Critical aspects of electrical connecter contacts'; erschienen in: International Conference on Electrical Contacts <21, 2002, Zürich> Proceedings / 21th International Conference on Electrical Contacts : 9 - 12 September 2002, Zurich, Switzerland / [Schweizerischer Elektrotechnischer Verein. Ed. by Werner Johler]. - Fehraltorf : SEV, 2002. - VIII S., S. 19-555, ISBN: 3-9522504-0-6 Text zu finden unter http://www.pvdijk.com/pdf/21thiceccriticalaspects.pdf, gesehen am 27.10.2006; und J.W. McBright und C. Maul, 'Intermittency events in bio-compartible electrical contacts', in: "Electrical Contacts, 2005, Proceedings on the 51 st Holm Conference, Seite 75-81, ISBN 0-7803-9113-6). In der genannten Literatur wird beschrieben, dass bei einer Normalkraft von 1N ein a-spot-Durchmesser von maximal 75 µm zu erwarten ist. Die elektrische Kontaktierung erfolgt daher über metallische Kontaktpunkte mit einer Größe von etwa 10 bis 20 µm Durchmesser. Um die auf den Kontaktflächen angeordneten Deckschichten zu entfernen, sind Normalkräfte von etwa 1 N erforderlich. Für die axiale Führung des zylindrischen Steckers sind mindestens drei - möglichst gleichmäßig über den Umfang verteilte - Berührungspunkte erforderlich. In diesem Fall kann davon ausgegangen werden, dass die drei Kontaktkräfte von gleichem Betrag sind. Sind konstruktiv mehr als drei Berührungspunkte (Kontaktstellen) vorgesehen, so kann es aufgrund von Herstellungs- und Fertigungstoleranzen zu einer ungleichmäßigen Verteilung der Normalkräfte in den Berührungspunkten kommen, so dass nicht davon ausgegangen werden kann, dass in allen Berührungspunkten die Deckschichten wirksam durchstoßen werden können. Durch die erfindungsgemäße Vielzahl flexibler Kontaktfasern werden diese Nachteile behoben, indem durch die Vielzahl der flexiblen Fasern eine in weiten Bereichen konstante Normalkraft in den Berührungspunkten eingestellt werden kann.

Die erfindungsgemäße Kontaktanordnung hat den Vorteil, dass die Kontaktflächen auf die "wahren" Kontaktflächen (a-spots) reduziert sind und hierdurch ein hoher Grad an Redundanz bei der Kontaktierung und eine höhere Kontaktstabilität unter mechanischer Last erreicht wird. Die erfindungsgemäße Kontaktanordnung, die somit auf das Wesentliche, nämlich die wirksamen Kontaktflächen, reduziert ist, ist außerdem deshalb vorteilhaft, da aufgrund ihrer Gestaltung eine Minimierung des Einflusses der konstruktiv beeinflussbaren Kenngrößen, des Einflusses von Werkstoffparametern und des Einflusses von Prozessparametern bei der Herstellung erfolgt sowie die benötigten Werkstoffe auf dem Markt eine gute Verfügbarkeit besitzen. Mit der erfindungsgemäßen Kontaktanordnung wird zudem Spaltkorrosion verhindert.

Besonders bevorzugt besteht die erfindungsgemäße Kontaktanordnung aus einer flexiblen Fasermatte (Faserbahn) mit einer vorgegebenen Dicke. Vorzugsweise liegt die Dicke der Fasermatte zwischen (einschließlich) 0,5 und 1,5 mm, besonders bevorzugt zwischen (einschließlich) 0,7 und 1,2 mm. Derartige flexible Matten werden mittels herkömmlicher, klassischer Verarbeitungsverfahren der Textilindustrie als Gewebe, Gestricke oder Gewirke hergestellt und weisen beispielsweise ein geordnetes Gewebe auf. Die Fasern mit metallischer Leitfähigkeit können beispielsweise auch zu einem Vlies mit sogenannten Wirrfasern, d.h. ungeordnet angeordneten Fasern, mit gleichmäßiger Flächendichte vervliest werden.

Vorzugsweise ist die Kontaktanordnung für den Buchsenkontaktabschnitt mit einer in Längsrichtung der Buchse verlaufenden durchgehenden Öffnung versehen, die einen im Wesentlichen runden Querschnitt aufweist. Eine derartige Öffnung ist besonders einfach herstellbar. Die Öffnung weist bevorzugt einen mittleren Durchmesser DO auf, der kleiner als der Durchmesser des Steckerkontaktabschnitts ist, der zur Anordnung in der Öffnung im Bereich der Kontaktanordnung bestimmt ist. Hierdurch werden die erforderlichen Normalkräfte zwischen Kontaktelement und Gegenkontakt am Stecker hergestellt. Besonders bevorzugt ist der mittlere Durchmesse DO der durchgehenden Öffnung um mindestens 0,3% und höchstens 3,5% des Durchmessers des Steckerkontaktabschnitts kleiner als der Durchmesser des Steckerkontaktabschnitts.

In einem besonders bevorzugten Ausführungsbeispiel weist die Öffnung in ihrem Umfang einen oder mehrere Schlitze, vorzugsweise drei Schlitze, auf, welche im Abstand von etwa 120° angeordnet sind. Hierdurch wird die Kontaktstabilität einerseits und andererseits die Steckbarkeit der Kontaktanordnung gewährleistet. Die Schlitzbreite und -anordnung und damit die Normalkräfte können individuell eingestellt werden. Besonders bevorzugt beträgt die Schlitzbreite zwischen 10% und 40% des Umfangs der durchgehenden Öffnung.

In weiteren Ausführungsbeispielen kann die durchgehende Öffnung auch andere Querschnittsformen, beispielsweise eine Fünf- oder Sechseckform aufweisen. Analog zur Ausführungsform mit dem runden Querschnitt können in derartigen Kontaktelementen Schlitze eingefügt sein oder die Breite der Öffnung entsprechend kleiner als der Durchmesser des entsprechenden Steckerabschnitts gestaltet sein.

In einem weiteren bevorzugten Ausführungsbeispiel besteht das Kontaktelement für den Steckerkontaktabschnitt aus dem gleichen Material wie das Kontaktelement für den Buchsenkontaktabschnitt, vorzugsweise aus einem mattenförmigen Fasergeflecht, welches einen im Wesentlichen kreisförmigen Querschnitt aufweist. Zur Realisierung der erforderlichen Mindestandruckkraft weist das Fasergeflecht in seinem Querschnitt einen mittleren Durchmesser DK auf, der größer als der Durchmesser der Öffnung des Buchsenkontaktabschnitts ist, welcher zur Anordnung des Steckerkontaktabschnitts im Bereich der Kontaktanordnung bestimmt ist. Besonders bevorzugt ist der mittlere Durchmesser DK des Fasergeflechts um mindestens 0,3% und höchstens 3,5% des Durchmessers der Öffnung größer als der Durchmesser der Öffnung.

Besonders einfach können die erfindungsgemäßen Kontaktanordnungen dadurch hergestellt werden, dass die durchgehende Öffnung des Kontaktelements des Buchsenkontaktabschnitts bzw. die Außenkante oder die Außenkanten des Kontaktelements des Steckerkontaktabschnitts mittels Laserschneiden oder mittels mechanischem Stanzen hergestellt sind. Beim Laserschneiden lässt sich die Energie des Lasers hierbei so einstellen, dass in einem besonders bevorzugten Ausführungsbeispiel mindestens ein Teil der Vielzahl von Fasern des Fasergeflechts mit einem abgerundeten Ende in die Öffnung ragt bzw. von der Mantelfläche nach außen wegragt. Dadurch wird die Oberflächenrauigkeit vorteilhaft minimiert.

Besonders im Fall der Kontaktanordnung für einen Buchsenkontaktabschnitt kann die Kontaktanordnung ein Halteelement in Form eines Rahmens aus einem metallischen Material aufweisen, in den das Fasergeflecht in eingefasst ist. Eine derartige Kontaktanordnung besitzt den Vorteil, dass sie einfach herstellbar ist.

An seinen Außenkanten, besonders im Fall des Kontaktelements für einen Buchsenkontaktabschnitt, können die Fasern des Fasergeflechts mittels eines thermischen Verfahrens, beispielsweise mittels Laser, fixiert sein.

Der mittlere Durchmesser d der Fasern beträgt maximal 75 µm, vorzugsweise 2 ≤ d ≤ 50 µm, weiter bevorzugt 10 ≤ d ≤ 50 µm, insbesondere bevorzugt 10 ≤ d ≤ 35 µm. Mittels diesen Faserdurchmessern wird der Faserdurchmesser vorteilhaft auf die Größe der jeweiligen a-spots, über die der elektrische Kontakt wirksam ist, reduziert.

Besonders geeignete Materialien im Hinblick auf die Leitfähigkeit, Korrosionsbeständigkeit und Biokompatibilität kommen zum Einsatz, wenn die Fasern des Fasergeflechts mindestens teilweise aus einem Material der Gruppe Platin, Iridium, Edelstahl gem. einschlägiger Normen über Implantatwerkstoffe (ISO; ASTM), bevorzugt Edelstahl 316L oder Edelstahl 316L-VM, Titan-Legierung, leitfähiger Kunststoff, Kohlefaser, leitfähige Keramik oder einer Mischung aus mindestens zwei Materialien dieser Gruppe besteht. Der Füllgrad des Fasergeflechts liegt vorteilhaft zwischen (einschließlich) 50% und 80%, bevorzugt zwischen (einschließlich) 50% und 70%.

Besonders bevorzugt sind mindestens 60 Kontaktpunkte mittels der in die durchgehende Öffnung hineinragenden oder von der Mantelfläche wegragenden Fasern des Fasergeflechts mit dem Steckerkontaktabschnitt bzw. dem Buchsenkontaktabschnitt herstellbar.

Bei der Herstellung der Kontaktanordnung gut handhabbar ist das Fasergeflecht, wenn es mit Silikon oder einem flexiblen elektrisch isolierenden Kunststoff verfüllt ist. Zusätzlich wird die Ausbreitung von Körperflüssigkeiten entlang der Stecker- und Buchsen-Längsachse wirksam verhindert.

Die obige Aufgabe wird erfindungsgemäß auch durch eine Kontaktbaugruppe für einen Stecker oder eine Buchse mit einem oder mehreren erfindungsgemäßen Kontaktanordnungen gelöst. Die erfindungsgemäße Kontaktbaugruppe schafft einen hohen Grad an Redundanz bei der Kontaktierung und erhöht die Kontaktstabilität unter mechanischer Last. In einem besonders bevorzugten Ausführungsbeispiel weist eine Kontaktbaugruppe mit mehreren Kontaktanordnungen zwischen diesen eine elektrisch isolierende Anordnung auf, um Fehler bei der Stromleitung zu vermeiden und die einzelnen Kontaktanordnungen voneinander zu isolieren.

In einem weiteren bevorzugten Ausführungsbeispiel weist die Kontaktbaugruppe auch eine Dichtanordnung auf, welche zur Abdichtung der implantierbaren Vorrichtung gegenüber Körperflüssigkeiten dient.

Eine Kontaktbaugruppe mit mehreren Kontaktanordnungen weist einen Abstand I der Mitten der Kontaktelemente zweier benachbarter Kontaktanordnungen in axialer Richtung der Kontaktbaugruppe einen Abstand zwischen (einschließlich) 2 und 6 mm, vorzugsweise zwischen (einschließlich) 3 und 5 mm, besonders bevorzugt zwischen (einschließlich) 4 und 5 mm auf. Durch diese Gestaltung der Kontaktbaugruppe wird die Norm für mehrpolige Stecker und Steckerbuchsen, beispielsweise die IS-4-Norm erfüllt.

Die obige Aufgabe wird außerdem erfindungsgemäß durch eine implantierbare Vorrichtung mit einer erfindungsgemäßen Buchsen-Kontaktbaugruppe und einer elektronischen Vorrichtung mit mindestens einem Anschluss gelöst, wobei der mindestens eine Anschluss der elektrischen Vorrichtung mit einer Kontaktanordnung elektrisch leitend verbunden ist. Entsprechend wird die obige Aufgabe erfindungsgemäß auch durch eine Elektrodenleitung mit einem an einem Ende angeordneten Stecker mit einer erfindungsgemäßen Stecker-Kontaktbaugruppe gelöst, wobei die Elektrode elektrisch leitend mit mindestens einer der Kontaktanordnungen verbunden ist. Die erfindungsgemäße implantierbare Vorrichtung, beispielsweise ein Herzschrittmacher, ein Defibrillator, eine neurologische Vorrichtung oder eine Muskelstimulierungsvorrichtung, und die erfindungsgemäße Elektrodenleitung erlauben die Herstellung von zuverlässigen elektrischen Kontakten, welche unter mechanischer Last besonders stabil sind.

Weitere Ziele, Merkmale, Vorteile und Anwendungsmöglichkeiten der Erfindung ergeben sich aus der nachfolgenden Beschreibung von Ausführungsbeispielen und den Zeichnungen. Dabei bilden aller beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen schematisch:
- Fig. 1: eine erfindungsgemäße implantierbare Vorrichtung und einen Abschnitt einer erfindungsgemäßen Elektrodenleitung in einer perspektivischen Ansicht von der Seite,
- Fig. 2: einen Längsschnitt durch eine erfindungsgemäße Kontaktbaugruppe einer Buchse,
- Fig. 3: einen Ausschnitt aus dem Querschnitt gemäß Fig. 2,
- Fig. 4: ein Kontaktelement für eine erfindungsgemäße Kontaktanordnung für einen Buchsenkontaktabschnitt in einer Ansicht von oben,
- Fig. 5: einen Ausschnitt aus einem Schnitt durch das Kontaktelement gemäß Fig. 4,
- Fig. 6 und 7: weitere Ausführungsbeispiele von Kontaktelementen für eine erfindungsgemäße Kontaktanordnung eines Buchsenkontaktabschnitts,
- Fig. 8: ein Ausführungsbeispiel für eine erfindungsgemäße Kontaktbaugruppe für einen Stecker in einer Ansicht von der Seite und
- Fig. 9: ein Kontaktelement einer erfindungsgemäßen Kontaktanordnung der Kontaktbaugruppe gemäß Fig. 8.

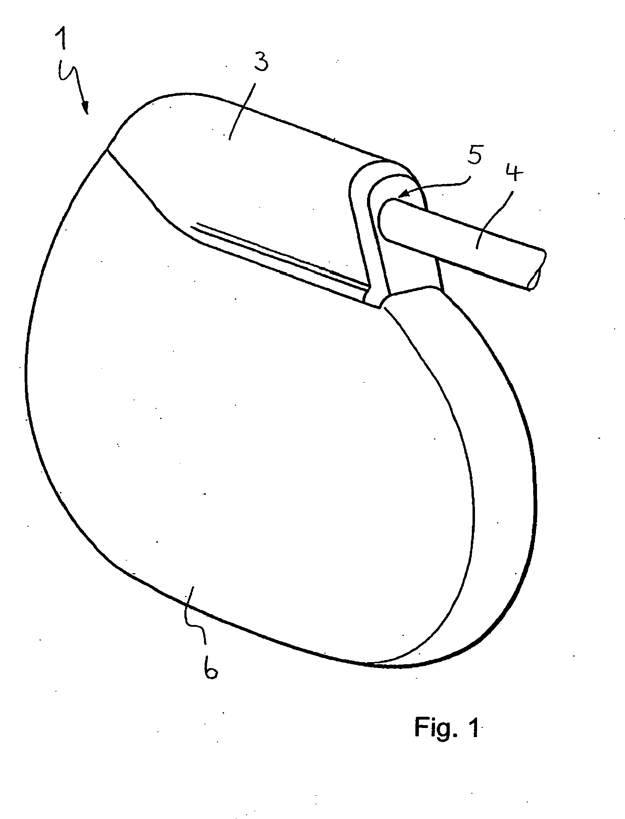

Fig. 1 zeigt einen Herzschrittmacher 1 mit einem Anschlussgehäuse (Header) 3 und einem Elektrodenleiter 4. Der Elektrodenleiter 4 ist mit seinem an einem Ende des Elektrodenleiters 4 angeordneten Stecker in der Buchse 5, welche als Steckbuchse ausgebildet ist, eingesteckt. Hierdurch ist die an dem nicht dargestellten, dem Stecker gegenüber liegenden Ende des Elektrodenleiters 4 angeordnete Elektrode elektrisch mit der im Gehäuse 6 angeordneten elektronischen Vorrichtung verbunden. In dem Gehäuse 6 befindet sich außerdem die Batterie des Herzschrittmachers, welche die elektronischen Vorrichtung, beispielsweise die elektronischen Platine, mit Spannung versorgt.

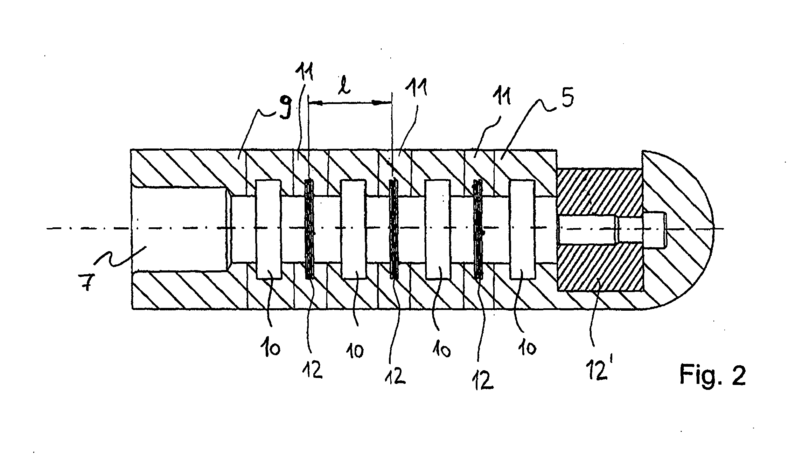

In Fig. 2 ist ein Längsschnitt durch eine als Buchse 5 ausgebildete Kontaktbaugruppe dargestellt. Die Buchse 5 weist eine durchgehende Öffnung 7 auf, welche mehrere Abschnitte mit unterschiedlichem, im Wesentlichen kreisförmigen Querschnitt (unterschiedlicher Durchmesser) aufweist. Die Öffnung 7 endet im vorderen Endbereich der Buchse 5. Die Buchse besteht in ihren die Öffnung 7 umgebenden Bereichen im Wesentlichen aus isolierendem Material 9, in dem Dichtungsanordnungen 10 und Kontaktanordnungen 11 abwechselnd hintereinander in Richtung der Längsachse angeordnet sind. In dem in Fig. 2 dargestellten Ausführungsbeispiel einer Buchse 5 sind drei Kontaktelemente 12 mit geringerer Dicke (Anordnung in Längsrichtung der Buchse 5) sowie ein weiteres, am vorderen Ende der Buchse angeordnetes breiteres Kontaktelement 12' hintereinander angeordnet. Jedes Kontaktelement ist dabei in einer Kontaktanordnung 11 angeordnet und in dieser mittels eines Halteelements festgelegt. Die dargestellte Buchse ist für einen 4-poligen Stecker bestimmt. Mit dem Buchstaben I ist in Fig. 2 der Abstand zweier Mitten der Kontaktelemente 12 in Richtung der Längsachse der Buchse 5 bezeichnet.

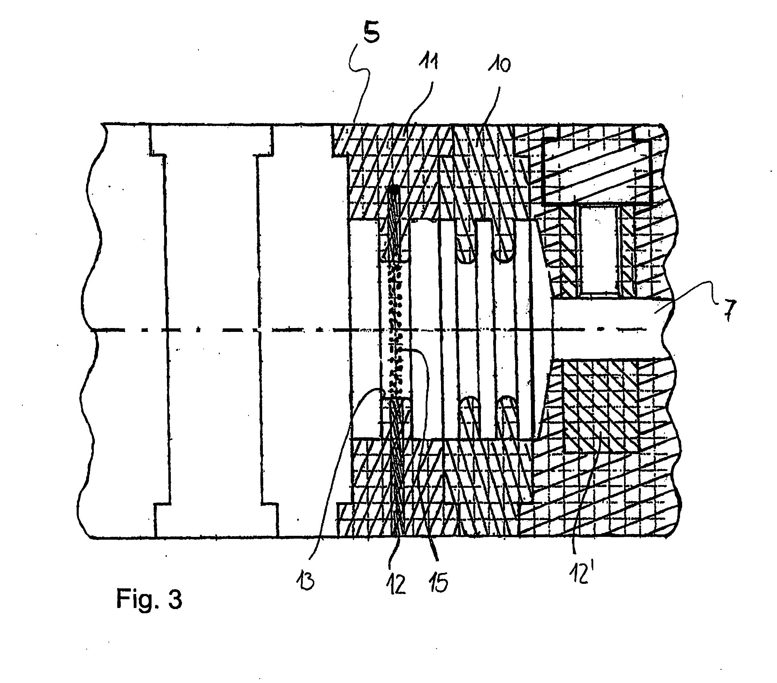

Anhand von Fig. 3 ist ein Ausschnitt der in Fig. 2 dargestellten Buchse 5 gezeigt. Bevor sich die Öffnung 7 in Richtung des vorderen Endes verjüngt, ist eine Dichtungsanordnung 10 als Segment der Buchse vorgesehen. Davor, in Richtung Einsteck-Öffnung der Buchse 5 ist eine Kontaktbaugruppe 11 mit einem mittig angeordneten Kontaktelement 12 in Form eines flexiblen Fasergeflechts (Matte) vorgesehen, welches eine kreisrunde Öffnung 13 aufweist. Die Enden der in die Öffnung hinein ragenden Fasern 15 des Fasergeflechts kommen beim Einstecken des Steckers mit diesem in Kontakt. Das Kontaktelement aus dem Fasergeflecht, das aus metallischen Fasern besteht, ist derart in der Kontaktanordnung 11 mittels des nicht dargestellten Halteelements beispielsweise in Form eines metallischen Rahmens gehalten, dass sie mit ihrer größten Ausdehnung in etwa senkrecht zur Längsachse der Buchse 5 verläuft.

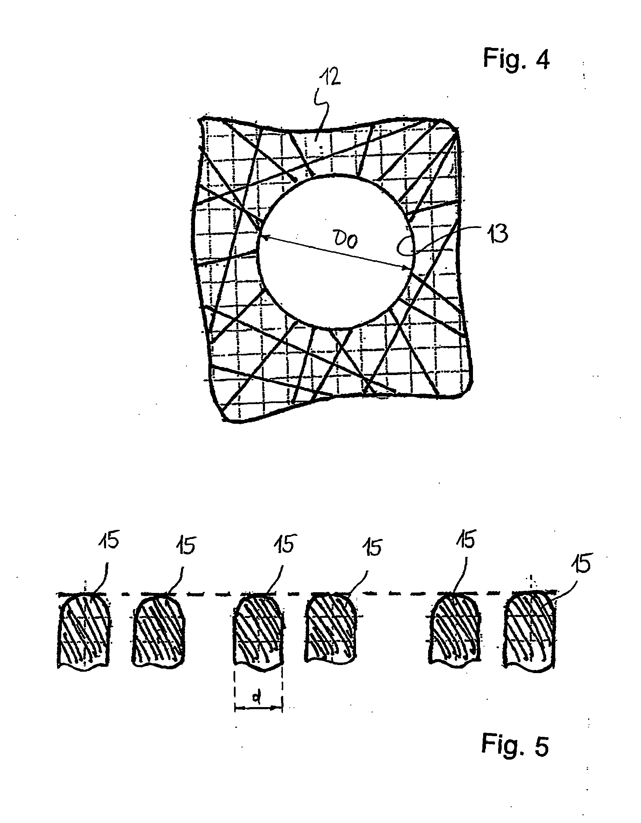

In Fig. 4 wird ein Ausschnitt des Kontaktelements 11 bestehend aus dem flexiblen Fasergeflecht unregelmäßig angeordneter Fasern 15 mit der kreisförmigen Öffnung 13 noch einmal separat dargestellt. Der mittlere Durchmesser DO der kreisförmigen Öffnung ist in der Darstellung eingezeichnet. Er erstreckt sich von einem Rand der kreisförmigen Öffnung 13 zu dem gegenüber liegenden Rand und verläuft durch den Mittelpunkt der Öffnung 13.

Fig. 5 zeigt einzelne Fasern 15 mit einem mittleren Durchmesser d, die in die Öffnung 13 hinein ragen und den mit einer gestrichelten Linie dargestellten Rand ausbilden. Es ist zu erkennen, dass die Fasern, die in die Öffnung hineinragen, ein abgerundetes Ende aufweisen. Dies kann erreicht werden, indem die Energie des Lasers, mit dem die Öffnung aus der Fasermatte herausgeschnitten wird, entsprechend eingestellt wird, dass das Material der Fasern anschmilzt oder erweicht.

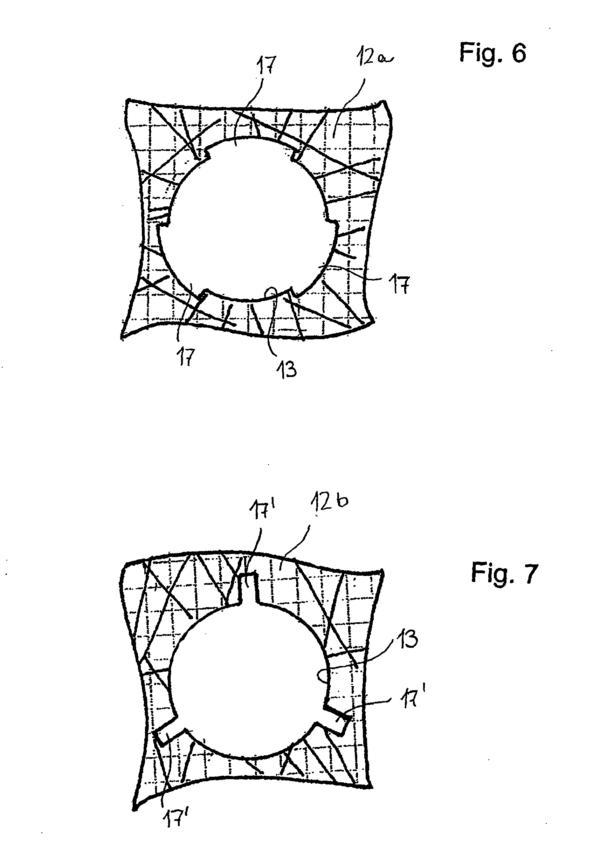

Anhand der Figuren 6 und 7 sind weitere Ausführungsbeispiele für Kontaktelemente 12a und 12b dargestellt. Die Kontaktelemente 12a und 12b weisen Schlitze 17 auf, welche in Umfangsrichtung eine unterschiedliche Länge aufweisen. Die Länge der drei Schlitze des Kontaktelements 12a in Fig. 6 beträgt insgesamt, verglichen mit dem Gesamtumfang der Öffnung 13 etwa die Hälfte des Gesamtumfangs. Die Schlitze sind etwa im Abstand von 120° bezogen auf ihre jeweiligen Mitte der Längsausdehnung auf dem Umfang der Öffnung angeordnet. Ebenfalls im Abstand von 120° sind die Schlitze 17' des Kontaktelements 12b in Fig. 7 vorgesehen. Diese haben eine, verglichen mit dem Ausführungsbeispiel in Fig. 6, eine deutlich geringere Ausdehnung in Richtung des Umfangs der Öffnung 13. Allerdings ist die Tiefe der Schlitze 17' in radialer Richtung größer als die Schlitze 17 des Ausführungsbeispiels in Fig. 6. Die Schlitze 17 können bis zur Einfassung des Kontaktelements 12a, 12b reichen. Die Schlitze 17, 17' verlaufen im Wesentlichen in radialer Richtung. Durch die Gestaltung der Schlitze kann die Flexibilität der Kontaktierung gegenüber mechanischen Störeinflüssen eingestellt werden; dadurch wird das "Atmen", d.h. das kurzzeitige Trennen der Kontaktstellen, und das Reiben der Kontaktstellen, das so genannte Fretting, unterbunden.

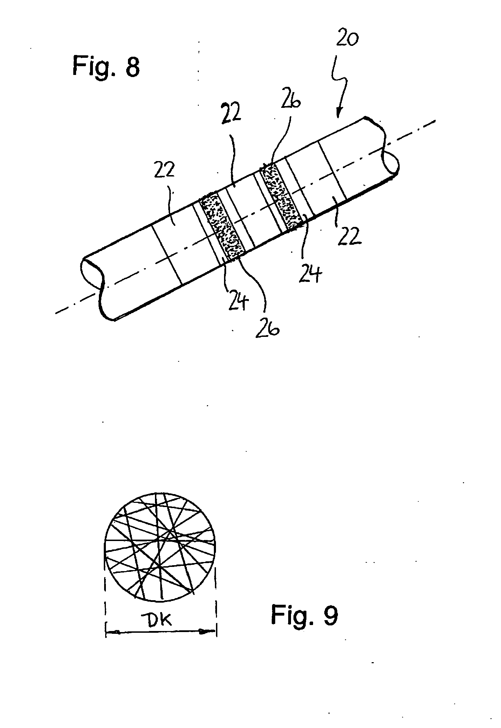

In Fig. 8 ist ein Abschnitt eines Steckers 20, der in eine Buchse 5 eingesteckt werden kann, dargestellt. Der Stecker 20 ist im Wesentlichen zylindrisch geformt und weist im Wechsel entlang seiner Längsachse isolierende Bereiche 22 und Kontaktanordnungen 24 auf. Jede Kontaktanordnung weist ein Kontaktelement 26 in Form einer Fasergeflecht-Matte mit einem im Wesentlichen kreisförmigen Querschnitt auf, das in einem nicht dargestellten Halteelement festgelegt ist. Die Fasern an den den Außenumfang bildenden Außenkanten des Kontaktelements 26 ragen von der Mantelfläche des Steckers 20 weg und ermöglichen den Kontakt mit entsprechenden Gegenkontaktflächen in einer Buchse. Das Kontaktelement 26 ist in einer Ansicht von oben in Fig. 9 noch einmal separat dargestellt. Ferner ist der mittlere Durchmesser DK eingezeichnet, der sich von einer Außenkante des Kontaktelements 26 bis zu der anderen Außenkante erstreckt und durch den Mittelpunkt des Kontaktelements 26 hindurch geht.

In einem besonderen Ausführungsbeispiel sind die Fasern der Matte mit einem flexiblen, elektrisch isolierenden Kunststoff oder Silikon verfüllt. Dies erhöht die Einschubkräfte und die Steifigkeit der Faserscheibe. Es wird so ein Bereich erzeugt, der die elektrische Kontaktierung in dem Bereich aufbaut und zusätzlich die Ausbreitung von Flüssigkeit in Richtung der Steckerlängsachse verhindert. Zur Verfüllung des Fasergeflechts mit Silikon wird dieses in eine Form gegeben. Mittels Spritzguss wird die gewünschte Form anschließend mit Silikon oder Elastomeren, z.B. Polyurethan aufgefüllt. Für das Freihalten der Öffnung kann ein Kern in der Öffnung angeordnet werden. Besonders bevorzugt kann der gesamte Stecker mittels Spritzguss hergestellt werden.

Überraschenderweise wird beim Einstecken des Steckers durch die vorstehenden Faserenden eine Verkrallung dieser in den jeweiligen Gegenkontakten erreicht, so dass eine Vorspannung erzeugt wird.

An den Außenkanten kann die Fasermatte mittels Laserlöten fixiert werden. Die erforderlichen Querschnittsformen der Fasermatten können auch mittels Fotoätzen hergestellt werden.

Mittels der erfindungsgemäßen Kontaktanordnung können auch für Defibrillatoren, die zeitweise mit Spannungen von etwa 750 V arbeiten, gute elektrische Kontakte hergestellt werden.

### Bezugszeichenliste:

- 1: Herzschrittmacher
- 3: Anschlussgehäuse (Header)
- 4: Elektrodenleiter
- 5: Buchse
- 6: Gehäuse
- 7: Öffnung
- 9: isolierender Bereich
- 10: Dichtungsanordnung
- 11: Kontaktanordnung
- 12, 12': Kontaktelement
- 12a, 12b: Kontaktelement
- 13: Öffnung des Kontaktelements 12
- 15: Faser
- 17, 17': Schlitz
- 20: Stecker
- 22: isolierender Bereich
- 24: Kontaktanordnung
- 26: Kontaktelement

## Patentansprüche

1. Kontaktanordnung zur Herstellung einer elektrisch leitenden Verbindung zwischen einem im Wesentlichen zylindrischen Stecker und einer im Wesentlichen hohlzylindrischen Buchse, vorzugsweise für die Verbindung einer Elektrode mit einer elektronischen Vorrichtung eines implantierbaren Geräts, wobei die Kontaktanordnung entweder einen Kontaktabschnitt des Steckers oder einen Kontaktabschnitt der Buchse bildet, wobei der Buchsenkontaktabschnitt eine Öffnung aufweist, in die der Stecker einführbar ist, wobei die Kontaktanordnung mindestens ein Kontaktelement aufweist, wobei das Kontaktelement aus einem Geflecht mit Fasern besteht, die metallische Leitfähigkeit besitzen, **dadurch gekennzeichnet, dass** mindestens ein Ende einer Vielzahl von Fasern des Fasergeflechts des Kontaktelements in die Öffnung ragt bzw. von der Mantelfläche nach außen wegragt.

2. Kontaktanordnung nach Anspruch 1, **dadurch gekennzeichnet, dass** das Kontaktelement aus einer Fasermatte mit einer vorgegebenen Dicke besteht.

3. Kontaktanordnung nach Anspruch 2, **dadurch gekennzeichnet, dass** die Fasermatte eine Dicke zwischen (einschließlich) 0,5 und 1,5 mm, vorzugsweise zwischen (einschließlich) 0,7 und 1,2 mm aufweist.

4. Kontaktanordnung nach einem der vorhergehenden Ansprüche, **dadurch gekennzeichnet, dass** die Kontaktanordnung für den Buchsenkontaktabschnitt mit einer in Längsrichtung der Buchse verlaufenden, durchgehenden Öffnung versehen ist, die einen im Wesentlichen runden Querschnitt aufweist.

5. Kontaktanordnung nach Anspruch 4, **dadurch gekennzeichnet, dass** die Öffnung einen mittleren Durchmesser DO aufweist, der kleiner als der Durchmesser des Steckerkontaktabschnitts ist, der zur Anordnung in der Öffnung im Bereich der Kontaktanordnung bestimmt ist.

6. Kontaktanordnung nach Anspruch 5, **dadurch gekennzeichnet, dass** der mittlere Durchmesser DO der durchgehenden Öffnung um mindestens 0,3% und höchstens 3,5% des Durchmessers des Steckerkontaktabschnitts kleiner als der Durchmesser des Steckerkontaktabschnitts ist.

7. Kontaktanordnung nach einem der Ansprüche 4 bis 6, **dadurch gekennzeichnet, dass** die Öffnung in ihrem Umfang einen oder mehrere Schlitze, vorzugsweise 3 Schlitze im Abstand von etwa 120°, aufweist.

8. Kontaktanordnung nach Anspruch 7, **dadurch gekennzeichnet, dass** die Schlitzbreite mindestens 10% bis 40% des Umfangs der Öffnung beträgt.

9. Kontaktanordnung nach einem der Ansprüche 1 bis 3, **dadurch gekennzeichnet, dass** die Kontaktanordnung für den Steckerkontaktabschnitt ein Kontaktelement in Form eines Fasergeflechts mit einem im Wesentlichen kreisförmigen Querschnitt aufweist.

10. Kontaktanordnung nach Anspruch 9, **dadurch gekennzeichnet, dass** der Querschnitt des Fasergeflechts einen mittleren Durchmesser DK aufweist, der größer als der Durchmesser der Öffnung des Buchsenkontaktabschnitts ist, der zur Anordnung des Steckerkontaktabschnitt im Bereich der Kontaktanordnung bestimmt ist.

11. Kontaktanordnung nach Anspruch 10, **dadurch gekennzeichnet, dass** der mittlere Durchmesser DK des Fasergeflechts um mindestens 0,3% und höchstens 3,5% des Durchmessers der Öffnung größer als der Durchmesser der Öffnung ist.

12. Kontaktanordnung nach einem der vorhergehenden Ansprüche, **dadurch gekennzeichnet, dass** die Öffnung des Kontaktelements des Buchsenkontaktabschnitts bzw. die Außenkante oder die Außenkanten des Kontaktelements des Steckerkontaktabschnitts mittels Laserschneiden oder mittels mechanischem Stanzen hergestellt sind.

13. Kontaktanordnung nach einem der vorhergehenden Ansprüche, **dadurch gekennzeichnet, dass** mindestens ein Teil der Vielzahl von Fasern des Fasergeflechts mit einem abgerundeten Ende in die Öffnung ragt bzw. von der Mantelfläche nach außen wegragt.

14. Kontaktanordnung nach einem der vorhergehenden Ansprüche, **dadurch gekennzeichnet, dass** die Kontaktanordnung ein Halteelement in Form eines Rahmens aus einem metallischen Material aufweist, in den das Fasergeflecht eingefasst ist.

15. Kontaktanordnung nach einem der vorhergehenden Ansprüche, **dadurch gekennzeichnet, dass** die Fasern des Fasergeflechts an seinen Außenkanten mittels eines thermischen Verfahrens fixiert sind.

16. Kontaktanordnung nach einem der vorhergehenden Ansprüche, **dadurch gekennzeichnet, dass** der mittlere Durchmesser d der Fasern maximal 75 µm beträgt, vorzugsweise 2 ≤ d ≤ 50 µm, weiter bevorzugt 10 ≤ d ≤ 50 µm, insbesondere bevorzugt 10 ≤ d ≤ 35 µm.

17. Kontaktanordnung nach einem der vorhergehenden Ansprüche, **dadurch gekennzeichnet, dass** die Fasern des Fasergeflechts mindestens teilweise aus einem Material der Gruppe Platin, Iridium, Edelstahl gemäß einschlägiger Normen über Implantatwerkstoffe (ISO; ASTM), vorzugsweise Edelstahl 316L oder Edelstahl 316L-VM, Titan-Legierung, leitfähiger Kunststoff, Kohlefaser, leitfähige Keramik oder einer Mischung aus mindestens zwei Materialien dieser Gruppe besteht.

18. Kontaktanordnung nach einem der vorhergehenden Ansprüche, **dadurch gekennzeichnet, dass** der Füllgrad des Fasergeflechts zwischen (einschließlich) 50% und 80%, bevorzugt zwischen (einschließlich) 50% und 70% liegt.

19. Kontaktanordnung nach einem der vorhergehenden Ansprüche, **dadurch gekennzeichnet, dass** mindestens 60 Kontaktpunkte mittels der in die Öffnung hineinragenden oder von der Mantelfläche wegragenden Fasern des Fasergeflechts mit dem Steckerkontaktabschnitt bzw. dem Buchsenkontaktabschnitt herstellbar sind.

20. Kontaktanordnung nach einem der vorhergehenden Ansprüche, **dadurch gekennzeichnet, dass** das Fasergeflecht mit Silikon oder einem flexiblen, elektrisch isolierenden Kunststoff verfüllt ist.

21. Kontaktbaugruppe für einen Stecker oder eine Buchse mit einem oder mehreren Kontaktanordnungen nach einem der vorhergehenden Ansprüche.

22. Kontaktbaugruppe nach Anspruch 21 mit mehreren Kontaktanordnungen, **dadurch gekennzeichnet, dass** zwischen zwei Kontaktanordnungen eine elektrisch isolierende Anordnung vorgesehen ist.

23. Kontaktbaugruppe nach einem der Ansprüche 21 oder 22, **dadurch gekennzeichnet, dass** die Kontaktbaugruppe eine Dichtanordnung aufweist.

24. Kontaktbaugruppe nach einem der Ansprüche 21 bis 23 mit mehreren Kontaktanordnungen, **dadurch gekennzeichnet, dass** der Abstand der Mitten der Kontaktelemente zweier benachbarter Kontaktanordnungen in axialer Richtung bezogen auf die Kontaktbaugruppe zwischen (einschließlich) 2 und 6 mm, vorzugsweise zwischen (einschließlich) 3 und 5 mm, besonders bevorzugt zwischen (einschließlich) 4 und 5 mm beträgt.

25. Implantierbare Vorrichtung mit einer Buchsen-Kontaktbaugruppe nach einem der Ansprüche 21 bis 24 und einer elektronischen Vorrichtung mit mindestens einem Anschluss, wobei der mindestens eine Anschluss der elektronischen Vorrichtung mit einer Kontaktanordnung elektrisch leitend verbunden ist.

26. Elektrodenleitung mit einem an einem Ende angeordneten Stecker mit einer Stecker-Kontaktbaugruppe nach einem der Ansprüche 21 bis 24, wobei die Elektrode elektrisch leitend mit einer Kontaktanordnung verbunden ist.
